# FASCICULE DE BREVET EUROPEEN

(11) **EP 2 292 150 B1**
(45) Date de publication et mention de la délivrance du brevet: **15.11.2017**
(21) Numéro de dépôt: 10175114.7
(22) Date de dépôt: 02.09.2010
(51) Int. Cl.: A61B 17/06

(54) **Dispositif pour le traitement de l'incontinence urinaire.**
Vorrichtung zur Behandlung von Harninkontinenz
Device for treating urinary incontinence

(30) Priorité: 02.09.2009 FR 0955971
(43) Date de publication de la demande: 09.03.2011
(73) Titulaire: Cousin Biotech, 59117 Wervicq Sud (FR)
(72) Inventeur: Dalle, Valery, 59170, CROIX (FR); Solecki, Gilles, 59390, LANNOY (FR)
(74) Mandataire: Balesta, Pierre

(56) Documents cités:
- US-A1- 2005 131 391

## Description

L'objet de la présente invention se situe dans le domaine du traitement de l'incontinence urinaire féminine, associée ou non à un prolapsus.

Plus précisément, l'objet de la présente invention se situe dans le domaine du traitement de la cystocèle, cette cystocèle provoquant, outre des pesanteurs pelviennes, des troubles urinaires tels que l'incontinence urinaire.

L'objet de la présente porte donc sur un dispositif qui comprend, d'une part, une aiguille d'introduction et, d'autre part, un élément de soutènement incluant une bandelette et des moyens de fixation aptes à fixer cet élément de soutènement à l'aiguille d'introduction.

Un tel dispositif est connu notamment par les documents FR 2.843.014, FR 2.814.939, ainsi que par le document EP 1.342.450.

Dans le document FR 2.843.014, l'élément de soutènement est une bandelette dont chaque extrémité est prolongée par un fil de traction. Dans ce document, la fixation de la bandelette à l'aiguille se fait en introduisant le fil de traction dans le chas prévu à l'extrémité distale de l'aiguille. Lors de l'intervention chirurgicale, cette opération consistant à introduire l'extrémité du fil de traction dans le chas de l'aiguille peut s'avérer délicate ; de plus, un risque existe que le fil de traction puisse s'échapper du chas de l'aiguille lors de la manipulation du dispositif.

Dans le document FR 2.814.939, l'élément de soutènement consiste en une bande composite qui comprend une gaine aplatie de protection à l'intérieur de laquelle est disposée la bandelette. Dans ce document, la fixation de la bande composite sur l'aiguille est réalisée grâce à des moyens de connexion, aptes à être verrouillés et/ou déverrouillés. Ces moyens de connexion sont disposés, d'une part, sur chacune des deux extrémités de la bande composite et, d'autre part, sur le talon proximal de l'aiguille. Il s'agit en l'occurrence de deux éléments, l'un mâle et l'autre femelle, qui sont encliquetables l'un dans l'autre. Dans l'exemple de réalisation décrit dans ce document FR 2.814.939, le talon proximal de l'aiguille est spatulé et chaque embout de la gaine est aplati et comprend une fente adaptée pour la pénétration du talon et pourvue d'un moyen de retenue apte à être verrouillé et/ou déverrouillé manuellement.

Ainsi, avec un élément de soutènement selon ce document, pour la fixation de la bande composite sur l'aiguille, le praticien doit introduire le talon proximal de l'aiguille dans la fente prévue dans l'embout de la gaine aplatie jusqu'à encliquetage du moyen de retenue du talon.

Ultérieurement, lorsqu'il s'agit de désolidariser l'aiguille de la bande composite, le praticien doit déverrouiller manuellement le moyen de retenu : Aucune indication n'est donnée dans le document FR 2.814.939 sur la manière avec laquelle le praticien doit désencliqueter le talon proximal de l'aiguille de l'embout de la bande composite.

La demanderesse observe qu'à l'évidence les opérations de fixation et de déverrouillage peuvent s'avérer délicates pour le praticien, avec notamment le risque que le désencliquetage ne puisse être obtenu dans des conditions qui soient satisfaisantes pour la tranquillité et la sécurité du praticien.

Dans le document EP 1.342.450, les moyens de solidarisation entre l'aiguille et la bandelette sont constitués d'une part, par une rainure circulaire pratiquée autour de l'aiguille et, d'autre part, par un manchon creux rigide dans lequel est enfilée l'aiguille, ce manchon étant conformé pour venir s'encliqueter dans la rainure circulaire. Dans ce document, la bandelette peut être fixée soit au niveau du talon de l'aiguille soit au niveau de son extrémité distale, c'est-à-dire à proximité immédiate de sa pointe. L'enfilage du manchon creux autour de l'aiguille passe par la pointe en question ; cette opération peut s'avérer risquée pour le praticien qui peut soit se blesser soit abimer le gant qu'il porte. De plus, la présence du manchon creux, enfilé autour de l'aiguille, crée localement une surépaisseur qui peut être gênante lors de l'intervention.

On connaît également le document US 2005/131391 qui divulgue un élément de soutènement présentant un connecteur pour faciliter le passage dans le plancher pelvien. Plus précisément, ce document divulgue la méthode de fixation du connecteur sur la section réduite de l'aiguille.

Dans ce document, la fente du connecteur est alignée de façon parallèle avec la section réduite de l'aiguille pour être ensuite emboîtée par l'opérateur. Cette technique de fixation est très complexe ; la demanderesse observe que ce genre de technique est voué à des échecs très fréquents.

En effet, les connecteurs utilisés en chirurgie urologique sont des pièces de très faibles dimensions ; elles présentent en général un diamètre de l'ordre de 3 à 5 millimètres pour une longueur voisine de l'ordre de 8 millimètres. Il est donc très difficile de les tenir en main et, de ce fait, il est très difficile de les emboîter sur l'aiguille en les positionnant de façon parallèle à l'aiguille sans avoir un phénomène de ripage du connecteur par rapport à l'aiguille.

De façon générale, l'emboîtement du connecteur se fait correctement et de manière reproductible si elle se fait dans un mouvement de rotation sans ripage du connecteur.

Or, ce phénomène de ripage se produit de façon très fréquente si l'on présente la fente du connecteur parallèlement à l'axe de l'aiguille, comme c'est le cas dans le document US 2005/131391.

Ce phénomène de ripage est systématique lorsque la fente du connecteur est relativement plus fine ou si l'épaisseur de la paroi du connecteur est plus importante, comme c'est le cas dans le document US 2005/131391.

Ainsi, avec un connecteur comme proposé dans ce document, on constate de façon très fréquente, voire systématique, le phénomène ripage évoqué ci-dessus.

Ceci est rédhibitoire pour une utilisation médicale : dans un bloc opératoire, le chirurgien doit pouvoir, avec des gangs chirurgicaux, fixer très facilement le connecteur. Or, la partie conique du connecteur proposé dans le document US 2005/131391 ne répond pas à ces contraintes : le phénomène de ripage ne sera pas évité même si le chirurgien emboîte le connecteur de manière sensiblement oblique à l'axe de l'aiguille, et en mettant en contact l'aiguille avec la partie de la fente située au niveau de la partie conique.

La demanderesse soumet donc que le système proposé dans ce document US 2005/131391 n'est pas conçu pour éviter ce phénomène de ripage, mais uniquement pour faciliter le passage du connecteur dans les tissus lors d'une intervention chirurgicale.

La demanderesse observe en outre, avec le système proposé dans ce document, un phénomène de retournement des bords de la fente vers l'intérieur du connecteur si la fente est présentée de façon parallèle ou de façon oblique à l'axe de l'aiguille.

Le but de la présente invention est donc de pallier aux différents inconvénients précités dans les systèmes connus, et notamment ceux relevés dans les documents FR 2.843.014, FR 2.814.939, EP 1.342.450 et US 2005/131391, en permettant de réaliser la fixation de l'élément de soutènement à l'aiguille d'introduction dans un dispositif pour le traitement de l'incontinence urinaire.

Ce but est parfaitement atteint par le dispositif selon la présente invention.

A cet effet, le dispositif selon la présente invention comprend notamment : a) une aiguille d'introduction, et b) un élément de soutènement comportant une bandelette et des moyens de fixation aptes à fixer l'élément de soutènement à l'aiguille d'introduction sur une zone longitudinale déterminée de celle-ci.

Avantageusement, les moyens de fixation selon la présente invention comprennent une pièce de fixation qui est solidaire de l'élément de soutènement et qui est constituée au moins partiellement dans un matériau élastiquement déformable.

Cette pièce de fixation présente en outre au moins une portion tubulaire ayant une ouverture longitudinale.

Ainsi, l'ouverture longitudinale est agencée en sorte que la portion tubulaire de cette pièce de fixation soit apte à venir s'emboîter sur la zone déterminée de l'aiguille par application à force de cette pièce selon son ouverture longitudinale sensiblement perpendiculairement ou obliquement par rapport à la direction longitudinale de l'aiguille.

De plus, la pièce de fixation comporte des premiers moyens d'arrêt, et l'aiguille comporte des seconds moyens d'arrêt.

Ces premiers et seconds moyens d'arrêt sont aptes à coopérer ensemble pour empêcher le coulissement de la pièce de fixation emboîtée sur la zone déterminée de l'aiguille.

Ainsi, selon la disposition particulière de la présente invention, la mise en place sur l'aiguille de la pièce de fixation se fait grâce à la déformation de la pièce de fixation consistant notamment en l'écartement des bords de l'ouverture longitudinale lors de l'application à force de la pièce de fixation.

Cette déformation, qui est rendue possible par le fait que la pièce de fixation est constituée au moins partiellement dans un matériau élastiquement déformable, est obtenue par une application de la pièce de fixation contre l'aiguille qui est sensiblement perpendiculaire ou en oblique.

De façon contraire, dans les documents FR 2.814.939 et EP 1.342.450, l'application nécessaire à la fixation de l'aiguille par rapport à l'élément de soutènement se fait toujours dans la direction longitudinale de l'aiguille.

Dans une variante de réalisation de la présente invention, l'aiguille comporte, dans la zone déterminée, un évidement au moins partiellement périphérique dont les bords proximal et distal constituent des seconds moyens d'arrêt pour les faces avant et arrière de la pièce de fixation. Dans cette variante, les faces avant et arrière de la pièce de fixation font office de premiers moyens d'arrêt.

Ainsi, c'est l'intégralité de la pièce de fixation qui est mise en butée à l'intérieur de l'évidement.

Dans un mode de réalisation de cette variante, l'épaisseur de la portion tubulaire de la pièce de fixation est sensiblement égale à la profondeur de l'évidement.

Cette disposition présente l'avantage d'éviter que la pièce de fixation forme une surépaisseur à la surface de l'aiguille.

Dans une variante de réalisation avantageuse de la présente invention, la pièce de fixation est constituée d'une seule portion tubulaire dont l'ouverture longitudinale s'étend sur toute la longueur de la portion.

Cette variante est particulièrement avantageuse dans la mesure où elle évite radicalement l'enfilage par la pointe de l'aiguille ; ceci évite ainsi tout risque d'accident lors de cet enfilage.

Dans un mode de réalisation de cette variante, la portion tubulaire présente une découpe en biseau, pratiquée selon les bords de l'ouverture longitudinale.

Dans ce mode, pour la mise en place de la pièce de fixation sur l'aiguille, le praticien place la pièce de fixation de telle sorte que la découpe en biseau vienne en appui longitudinal sur la zone déterminée de l'aiguille ; puis, il exerce une application en oblique de la portion tubulaire contre l'aiguille pour obtenir l'emboîtement recherché.

La découpe en biseau a pour effet d'augmenter progressivement la distance qui sépare les deux bords opposés de l'ouverture longitudinale, la distance la plus importante étant au niveau de l'extrémité de la portion tubulaire.

Grâce à cette disposition, on obtient une déformation facilitée et progressive de la portion tubulaire jusqu'à son emboîtement sur la zone déterminée de l'aiguille. De plus, la présence de cette découpe en biseau facilite le désemboîtement de la portion tubulaire lorsque cela est nécessaire au cours de l'intervention, le praticien pouvant prendre appui sur les deux bords en biseau de l'ouverture pour réaliser la déformation inverse de cette portion tubulaire.

Cet avantage est particulièrement significatif lorsque, lors de l'intervention par exemple, la portion tubulaire est bloquée dans un évidemment périphérique formé dans l'aiguille dont la profondeur est sensiblement également à l'épaisseur de la portion tubulaire.

Selon une autre variante de réalisation de la présente invention, la pièce de fixation comprend une première et une seconde portions tubulaires, toutes deux pourvues d'une ouverture longitudinale.

Dans cette variante, la seconde portion vient dans le prolongement de la première mais avec son ouverture longitudinale qui est diamétralement opposée à celle de la première portion ; dans ce cas, le dispositif selon la présente invention comporte, à la jonction entre ces deux portions tubulaires, un orifice formé en oblique et dimensionné pour permettre le passage ajusté de l'aiguille, en sorte que l'emboîtement de la pièce de fixation sur la zone déterminée de l'aiguille puisse se faire selon les deux portions tubulaires après pivotement de la pièce une fois l'aiguille introduite jusqu'à la zone déterminée.

Cette variante de réalisation présente l'inconvénient de nécessiter le passage de l'aiguille dans l'orifice formé à la jonction entre les deux portions tubulaires.

Elle présente cependant l'avantage d'éviter tout risque potentiel de désengagement involontaire de la pièce de fixation qui pourrait être dû par exemple à une mauvaise manipulation entraînant un accrochage inconsidéré d'un des bords de l'ouverture longitudinale, accrochage réalisant la déformation inverse de la portion tubulaire et le désemboîtement de la portion.

Avec les deux portions tubulaires dont les ouvertures longitudinales sont diamétralement opposées, ce risque est totalement exclu.

Avantageusement, le dispositif selon la présente invention comporte en outre un élément de positionnement constitué au moins partiellement dans un matériau, de préférence semi-rigide, élastiquement déformable dont une première portion couvre une zone de l'aiguille et dont la deuxième portion est en contact avec la portion supérieure de la pièce de fixation.

L'agencement de cet élément de positionnement avec l'aiguille et la pièce de fixation facilite l'emboîtement de la pièce de fixation avec l'aiguille.

Dans une première variante de réalisation, l'élément de positionnement consiste en une languette plate.

Dans une deuxième variante de réalisation, l'élément de positionnement consiste en une gouttière apte à couvrir au moins partiellement la zone de l'aiguille, et éventuellement la portion supérieure de la pièce de fixation, pour éviter le glissement relatif de la pièce de fixation par rapport à l'aiguille.

La présente invention sera mieux comprise à la lecture de la description détaillée qui suit et qui va décrire deux exemples de réalisation particuliers du dispositif pour le traitement de l'incontinence urinaire féminine

Ces deux exemples sont illustrés aux figures annexées à la présente dans lesquelles :
- la figure 1 est une vue schématique du dispositif de traitement de l'incontinence urinaire féminine comprenant une aiguille d'introduction sur une zone déterminée dans laquelle est placée par emboîtement une pièce de fixation selon le premier exemple de réalisation ;
- la figure 2 est une vue en perspective de la pièce de fixation de la figure 1 emboîtée sur une zone déterminée de l'aiguille ;
- les figures 3a à 3c et 4a à 4c illustrent les étapes permettant la mise en place de la pièce de fixation de la figure 2 sur l'aiguille ;
- la figure 5 est une vue en perspective d'une pièce de fixation selon un second exemple de réalisation ; et
- les figures 6a à 6c et 7a à 7c illustrent les étapes de mise en place de la pièce de fixation selon ce second exemple sur l'aiguille.

Le traitement de l'incontinence urinaire féminine nécessite l'implantation d'un élément de soutènement, généralement d'une bandelette, sous l'urètre dans le corps de la patiente.

Eventuellement, cette bandelette est associée à une plaque destinée à traiter le prolapsus en renforçant la paroi vaginale.

Il est connu d'implanter un tel élément de soutènement telle une bandelette dans le corps à l'aide de deux aiguilles recourbées dont une première extrémité pointue est libre et dont la seconde est reliée à une extrémité de l'élément de soutènement.

Après implantation de l'élément de soutènement et après développement des tissus fibreux dans cette dernière, l'urètre est maintenu dans une position par rapport au pubis permettant de résoudre le problème constaté.

L'aiguille est généralement associée à un ancillaire ou une poignée permettant de faciliter le guidage de l'aiguille lors de son insertion, laquelle peut être réalisée par voie sus-pubienne ou par voie trans-obturatrice.

Cette technique opératoire peut être réalisée sous anesthésie locale sans avoir à ouvrir la paroi abdominale.

La présente invention concerne spécifiquement, dans un tel dispositif de traitement, les moyens de fixation de la bandelette ou plus généralement de tout élément de soutènement de l'urètre sur l'aiguille d'introduction.

Ainsi, la présente invention concerne un tel dispositif 1 adapté à cet effet.

Plus particulièrement, le dispositif 1 selon la présente invention comporte une aiguille 2 qui est courbe avec un rayon de courbure qui varie avec la voie d'abord choisie.

Son extrémité distale 2a est pointue, et son extrémité proximale 2b est reliée à un ancillaire ou une poignée (non représentée ici).

Sur une zone déterminée 2c de cette aiguille 2 est fixé de manière amovible un élément de soutènement 3, qui dans l'exemple illustré est une simple bandelette.

Il s'agit notamment d'une bandelette textile réalisée en différents matériaux biocompatibles tels que en polypropylène monofilament tricoté ou non-tissé ou en polyéthylènetéréphtalate.

Elle peut notamment être constituée sur toute sa longueur de matériau résorbable tel que l'acide polyglycolique (PGA), l'acide poly-L-lactique (PLLA) ou le polydioxanone (PDS) ou encore en matériau semi-résorbable, associant au moins deux des matériaux cités ci-dessus permettant que la bandelette disparaisse au moins partiellement de l'organisme au bout d'une durée déterminée.

Cette bandelette peut également être imprégnée d'un matériau facilitant son passage dans les tissus, par exemple du diméthylsiloxane ou du collagène.

L'élément de soutènement 3 peut également associer une telle bandelette avec par exemple une plaque permettant d'assurer un traitement simultané du prolapsus et de l'incontinence, cette plaque étant accrochée sur la bandelette.

Il peut également comporter une gaine protectrice destinée à faciliter le passage de la bandelette sur les tissus.

L'élément de soutènement 3 peut également être une bande composite du type de celle décrite dans le document FR 2.814.939.

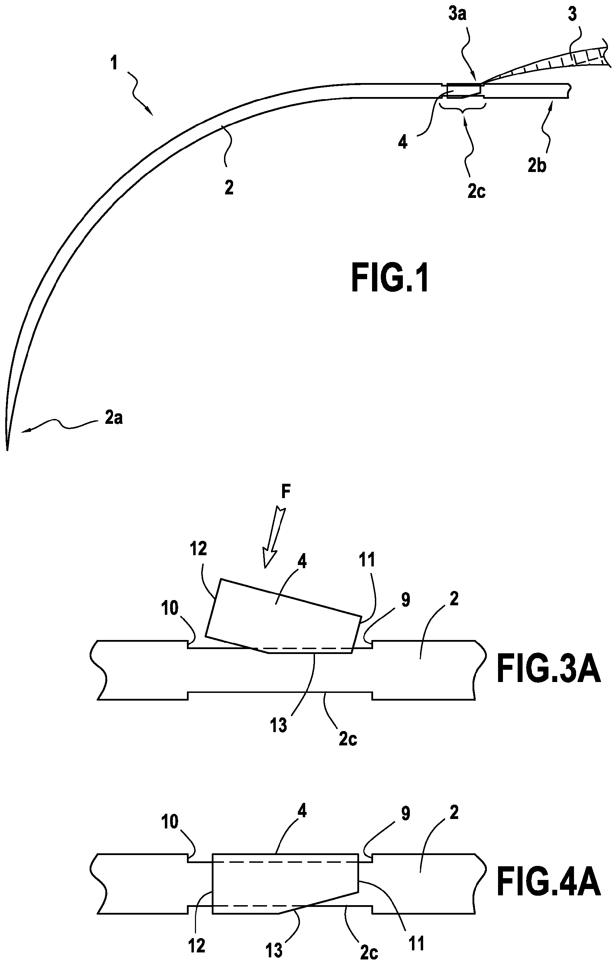

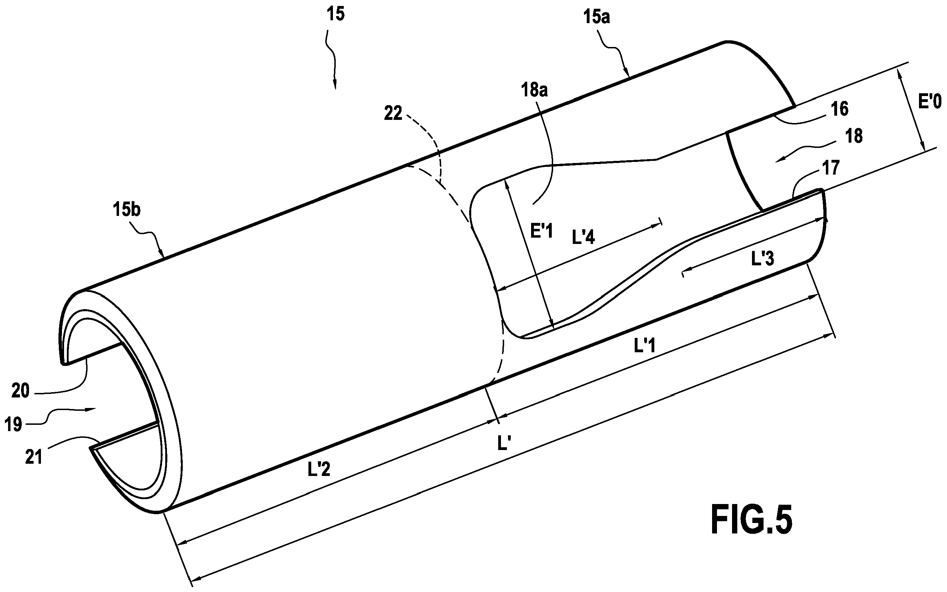

Selon la présente invention, l'élément de soutènement 3 est relié, à chacune de ses extrémités 3a à une pièce de fixation 4 ou 15 dont un premier exemple est décrit aux figures 1 à 4, et dont un second exemple est décrit aux figures 5 à 7.

Dans ces deux exemples, et de manière caractéristique, la pièce de fixation 4 ou 15 est constituée au moins partiellement dans un matériau élastiquement déformable, et qui présente au moins une portion tubulaire ayant une ouverture longitudinale en sorte que ladite portion tubulaire soit apte à venir s'emboîter sur la zone déterminée 2c de l'aiguille 2 par application à force de cette pièce 4 ou 15 selon son ouverture longitudinale.

Cette application est faite sensiblement perpendiculaire ou obliquement à l'aiguille 2.

Par ailleurs, cette application à force de la pièce de fixation 4 ou 15 contre l'aiguille 2, dans la zone déterminée 2c, doit provoquer l'écartement des deux bords opposés de l'ouverture longitudinale jusqu'à ce que cet écartement soit suffisant pour permettre le passage de l'aiguille 2.

Une fois que l'aiguille 2 est entrée dans la portion tubulaire, la force d'application est relâchée de sorte que du fait, du caractère élastique du matériau déformable, celui-ci reprend sa configuration initiale et la pièce de fixation 4 ou 15 enserre l'aiguille 2.

On comprend que la distance E0 entre les deux bords opposés 5 et 6 de l'ouverture longitudinale 7 pratiquée dans la portion tubulaire 8 de la pièce de fixation 4 doit être inférieure au diamètre D que présente l'aiguille 2 dans la zone déterminée 2c où doit être placée la pièce de fixation 4 ou 15.

Il est également préférable que l'emboîtement de la pièce de fixation 4 ou 15 sur l'aiguille 2 se face de la manière la plus ajustée possible.

Pour ce faire, il est souhaitable que le diamètre intérieur D1 de la portion tubulaire 8 de la pièce de fixation 4 soit sensiblement égal au diamètre D de l'aiguille 2 dans la zone déterminée 2c. La distance E0 entre les deux bords opposés 5 et 6 de l'ouverture longitudinale 7 est bien sûr fonction du diamètre D.

De manière générale, d'excellents résultats ont été obtenus avec une portion tubulaire qui, en section transversale, a la forme d'un arc de cercle d'angle au centre A faisant de 200° à 320°.

Le matériau déformable élastiquement pour la réalisation de la pièce de fixation 4 doit être bio-compatible : il peut s'agir de polypropylène, de polyéthylènetéréphtalate, de polyoxyméthylène, de polyamide 6, de polyamide 66, de polysulfone, de polyphénylsulfone ou de polyetheretherketone.

La fixation de l'extrémité 3a de l'élément de soutènement 3 sur la pièce de fixation 4 peut être obtenue par tout moyen adéquat, tout particulièrement sur la face extérieure 8a de la portion tubulaire 8 que ce soit par collage ou thermoliage.

Pour éviter que la pièce de fixation 4, une fois emboîtée sur le tube 2, puisse coulisser longitudinalement, le dispositif 1 selon la présente invention nécessite la mise en oeuvre de moyens d'arrêt à la fois sur la pièce de fixation 4 et sur l'aiguille 2, lesdits moyens d'arrêt étant aptes à coopérer ensemble pour empêcher ce coulissement.

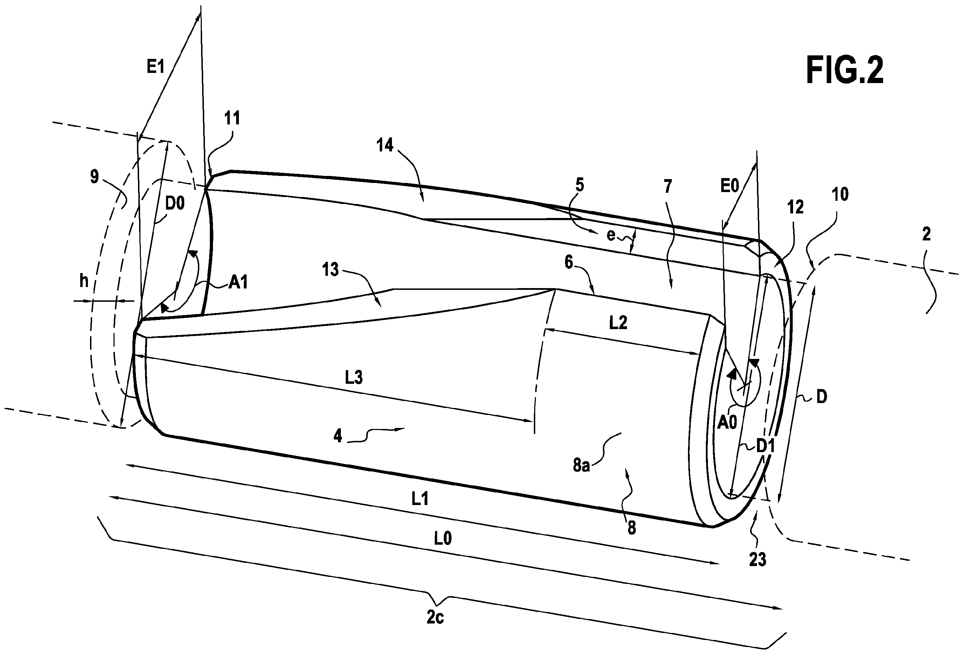

Dans l'exemple illustré à la figure 2, ces moyens d'arrêt consistent, s'agissant de l'aiguille 2, en un évidement périphérique pratiqué dans la zone déterminée 2c, cet évidement ayant pour effet de réduire, dans ladite zone 2c le diamètre D0 initial de l'aiguille 2 à la valeur D. Cette réduction de diamètre se traduit par la formation de deux faces annulaires 9,10 proximale et distale qui font office de butées pour les faces partiellement annulaires 11 et 12 d'extrémités de la pièce de fixation 4.

La distance L0 entre les deux faces annulaires 9 et 10 est bien sûr supérieure à la longueur L1 de la pièce de fixation 4 mais la différence entre ces deux longueurs L0 et L1 doit être la plus réduite possible de manière à éviter un libre coulissement de la pièce de fixation 4 le long de l'aiguille 2.

De plus, la profondeur de l'évidement 23, c'est-à-dire la hauteur h des faces annulaire 9 et 10 est sensiblement égale à l'épaisseur e de la portion tubulaire 8 de la pièce de fixation 4.

Ainsi, lorsque la pièce de fixation 4 est emboîtée sur l'aiguille 2 dans la zone déterminée 2c, la présence de cette pièce de fixation 4 ne crée pas de surépaisseur significative sur la longueur de l'aiguille.

Dans sa version la plus simple, non représentée, l'ouverture longitudinale 7 formée dans la portion tubulaire 8 présente la même largeur E0 entre ces deux bords opposés 5 et 6 sur toute la longueur L1 de la pièce de fixation 4.

Pour réaliser l'emboîtement de cette pièce de fixation sur l'aiguille, il faut que la distance E0 soit suffisamment grande par rapport au diamètre D de l'aiguille dans la zone déterminée 2c.

Il faut également exercer une pression importante pour obtenir la déformation de la pièce de fixation 4.

Pour éviter ces désagréments, la pièce de fixation 4 réalisée selon le premier exemple illustré comporte une découpe en biseau qui est réalisée selon les deux bords 5 et 6 de l'ouverture longitudinale 7.

Cette découpe a pour effet :
a) d'une part, d'augmenter progressivement la distance E0 entre les deux bords 5 et 6 de l'ouverture longitudinale 7 jusqu'à une valeur maximale E1, et
b) d'autre part, de diminuer l'angle au centre A0 jusqu'à une valeur minimale A1, lesdites valeurs maximales E1 et minimale A1 correspondant à la face 11 d'extrémité de la pièce de fixation.

De préférence, comme illustré sur la figure 2, la découpe en biseau n'est pas réalisée sur toute la longueur L1 de la pièce de fixation, mais seulement sur une partie L3 de celle-ci, la partie complémentaire de longueur L2 conservant les valeurs initiales E0 et A0. Les deux découpes en biseau 13,14 constituent des faces planes obliques.

Lors de la mise en place de la pièce de fixation 4 sur la zone déterminée 2c de l'aiguille 2, ce sont ces deux découpes en biseau 13 et 14 que le praticien vient tout d'abord appliquer sur l'aiguille 2, dans la zone déterminée 2c, à proximité de la face 9, comme illustré notamment à la figure 3a.

Puis, il exerce une pression, illustrée par la flèche F, jusqu'à obtenir l'emboîtement total de la pièce 4 sur l'aiguille 2 dans la zone déterminée 2c, comme illustré notamment à la figure 4a.

Du fait de la présence des découpes en biseau, la déformation de la portion tubulaire commence par se faire au niveau de l'extrémité correspondant à la face 11, ayant la distance entre les deux bords opposés E1 la plus importante et l'ange au sommet A1 le plus réduit jusqu'à remonter progressivement jusqu'à l'autre extrémité correspondant à la face 12.

Cette disposition préférée, d'une part, facilite la mise en place de la pièce de fixation et, d'autre part, permet de réduire la valeur E0 ce qui permet d'obtenir un blocage maximum de la pièce de fixation 4 sur l'aiguille 2.

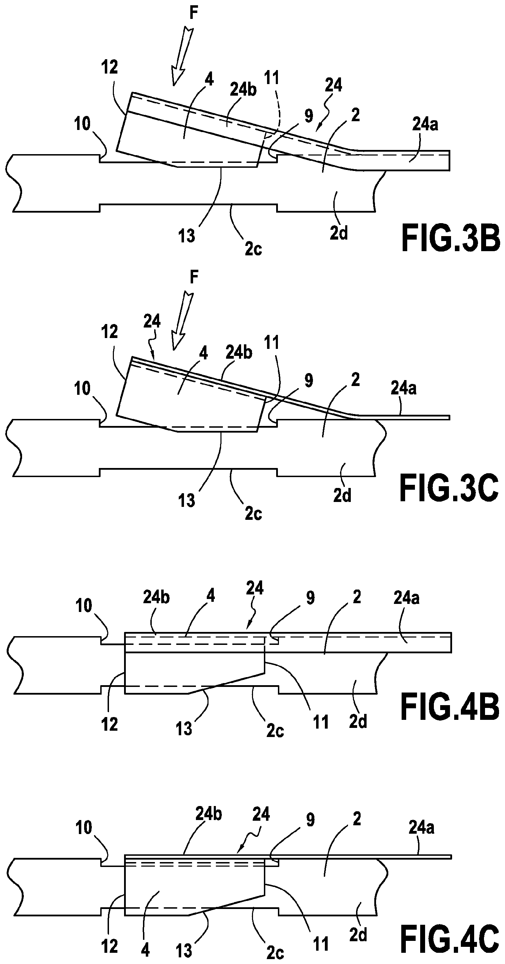

Avantageusement, et comme représenté notamment en figures 3b-4b et 3c-4c, le dispositif 1 selon la présente invention comporte en outre un élément de positionnement 24 qui est constitué au moins partiellement dans un matériau élastiquement déformable.

Cet élément de positionnement 24, qui est caractéristique de la présente invention, permet de faciliter l'emboîtement de la pièce de fixation 4 avec l'aiguille 2 ; ceci permet d'augmenter la précision de l'intervention.

Pour ce faire, cet élément le positionnement 24 est positionné en sorte qu'une première portion 24a de cet élément 24 couvre une zone 2d de l'aiguille 2, et que la deuxième portion 24b soit en contact avec la portion supérieure de la pièce de fixation 4.

Les caractéristiques du matériau élastiquement déformable ainsi que ce positionnement spécifique permettent ainsi de faciliter l'emboîtement de ladite pièce de fixation 4 avec l'aiguille 2.

Dans une première variante de réalisation illustrée en figures 3c et 4c, cet élément de positionnement 24 consiste en une languette plate.

Dans une deuxième variante de réalisation illustrée en figures 3b et 4b, cet élément de positionnement 24 consiste en une gouttière apte à couvrir au moins partiellement la zone 2d de l'aiguille 2 et la portion supérieure de la pièce de fixation 4, cette deuxième variante de réalisation permet avantageusement d'éviter le glissement relatif de la pièce de fixation 4 par rapport à l'aiguille 2.

De préférence, le matériau utilisé pour cet élément 24 présente des propriétés élastiques permettant d'avoir une force d'emboîtement inférieure à 40 newtons et une tenue dynamométrique de la pièce de fixation sur l'aiguille comprise entre 0 et 40 newtons pour un effort exercé de façon perpendiculaire à l'aiguille.

Le second exemple de réalisation va maintenant être décrit en référence aux figures 5 à 7.

Dans ce second exemple, la pièce de fixation 15 ne comporte pas une seule portion tubulaire ayant une ouverture longitudinale sur toute sa longueur comme dans le premier exemple, mais deux portions tubulaires 15a et 15b qui sont dans le prolongement l'une de l'autre et dont les ouvertures longitudinales 18 et 19 sont disposées diamétralement opposées l'une à l'autre.

Ces ouvertures 18 et 19 sont délimitées respectivement par les bords opposés 16 et 17 dans la première portion 15a et par les bords opposés 20 et 21 dans la seconde portion 15b.

Ainsi, l'ouverture longitudinale 18 de la première portion 15a se termine au niveau du plan faisant la jonction 22 avec la seconde portion tubulaire 15b.

Il en est de même inversement pour l'ouverture longitudinale 19 de la seconde portion 15b, laquelle se termine à la jonction 22.

De part et d'autre de cette jonction 22, les ouvertures longitudinales 18 et 19 sont aménagées, par découpe, en sorte d'augmenter localement la distance entre les deux bords opposés des ouvertures 18 et 19 en sorte de permettre un passage, en oblique, de l'aiguille 2.

Plus précisément, sur la figure 5, s'agissant de la première portion tubulaire 15a, la distance entre les deux bords opposés 16 et 17 de l'ouverture longitudinale 18, qui est de E'0 sur une première partie d'extrémité de longueur L'3, augmente ensuite sur la partie de longueur L'4 jusqu'à une valeur maximale E'1.

Cette valeur E'1 est déterminée en fonction du diamètre de l'aiguille 2.

Ceci est réalisé de la même manière mais de façon diamétralement opposée pour la seconde portion tubulaire 15b.

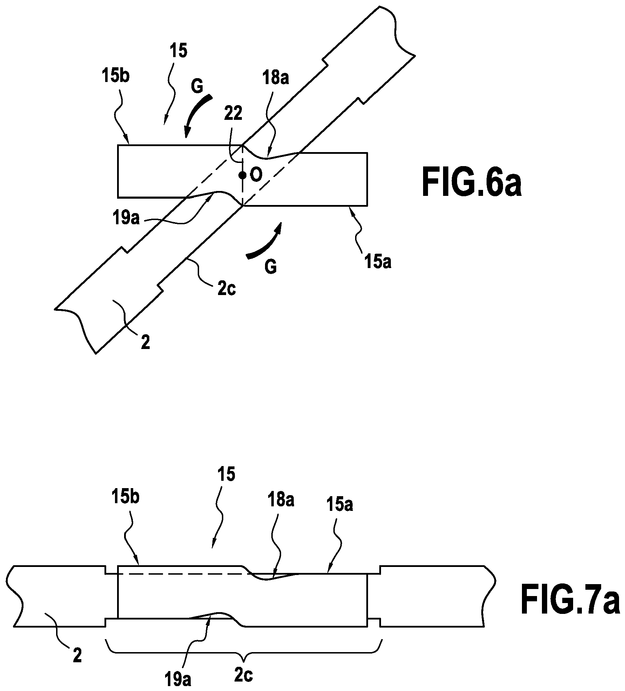

Sur les figures 6a et 7a, on a représenté la phase d'introduction de l'aiguille 2 dans les parties 18a et 19a des ouvertures longitudinales 18 et 19 des deux portions tubulaires 15a et 15b de la pièce de fixation 15 jusqu'à la zone déterminée 2c de l'aiguille 2.

L'emboîtement de la pièce de fixation 15 sur la zone déterminée 2c de l'aiguille 2 intervient en exerçant une pression sur la pièce de fixation 15 en sorte de faire pivoter celle-ci selon la flèche G par rapport à un axe fictif 0 qui se trouve dans le plan de la jonction 22.

Ainsi, l'emboîtement de la pièce de fixation 15 est obtenu grâce à l'écartement progressif des bords opposés 16-17 et 20-21 des deux ouvertures longitudinales 18 et 19 des deux portions tubulaires 15a et 15b.

Il est à noter que les découpes réalisées près de la zone de jonction 22 pour permettre le passage de l'aiguille 2 présente le même effet que les découpes en biseau du premier exemple en facilitant la déformation progressive de la pièce de fixation.

Pour désolidariser la pièce de fixation 15 de l'aiguille 2, le praticien doit obligatoirement réaliser le pivotement inverse des deux portions tubulaires 15a et 15b suivi du désenfilage de l'aiguille. Il ne peut donc y avoir en aucun cas un désemboîtement non souhaité par erreur de manipulation, comme cela pourrait éventuellement se produire avec la pièce de fixation 4 du premier exemple.

Pour une pièce de fixation 15 de longueur L', la première portion tubulaire 15a peut avoir une longueur L'1 qui est ou non identique à la longueur L'2 de la seconde portion tubulaire 15b.

A titre d'exemple, la première portion tubulaire 15a a une longueur L'1 qui est de deux à trois fois supérieure à la longueur L'2 de la seconde portion tubulaire.

Dans ce cas, le verrouillage le plus important de la pièce de fixation sur l'aiguille 2 se fait grâce à la partie de longueur L'3 de la première portion tubulaire 15a dans laquelle la distance entre les bords opposés 16 et 17 de l'ouverture longitudinale 18, E'0 est la plus faible.

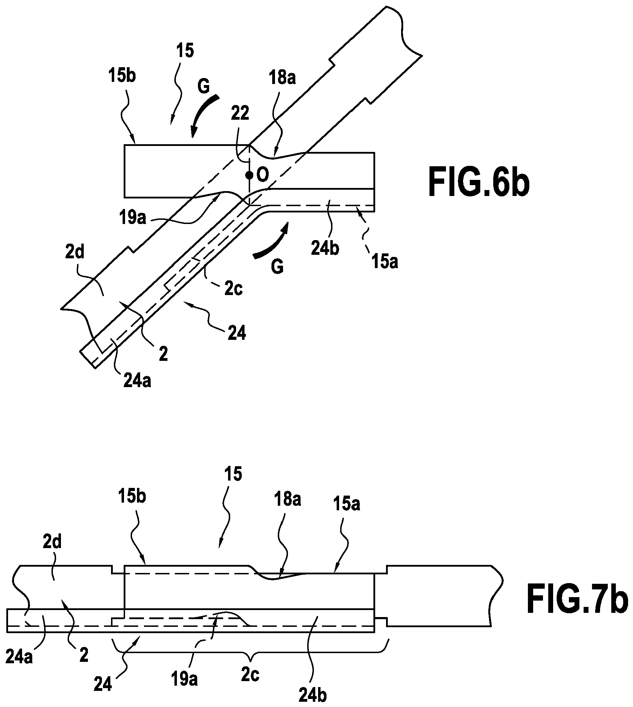

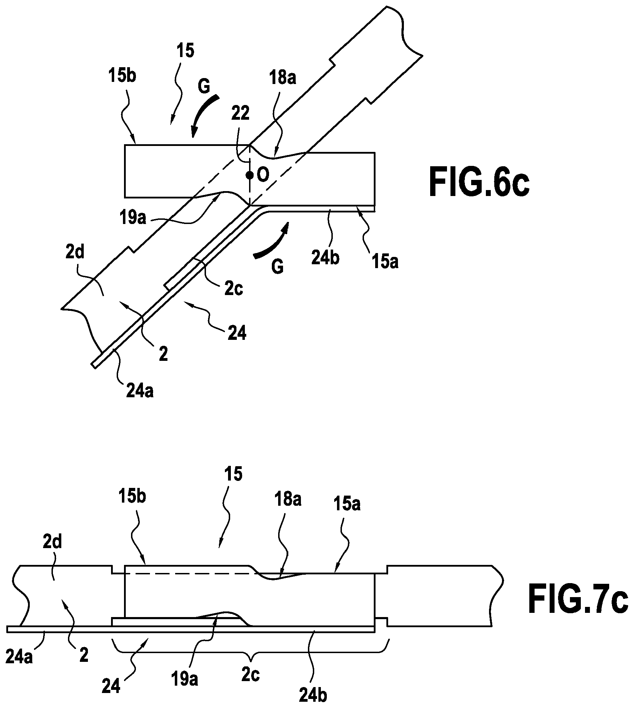

Avantageusement, et comme représenté notamment en figures 6b-7b et 6c-7c, le dispositif 1 selon la présente invention comporte en outre un élément de positionnement 24 qui est constitué au moins partiellement dans un matériau élastiquement déformable.

Le mode de fonctionnement de cet élément de positionnement est sensiblement identique à celui décrit ci-dessus pour le premier mode de réalisation.

De la même manière, cet élément de positionnement 24 permet de faciliter l'emboîtement de la pièce de fixation 15 avec l'aiguille 2.

Pour ce faire, cet élément le positionnement 24 est positionné en sorte qu'une première portion 24a de cet élément 24 couvre une zone 2d de l'aiguille 2, et que la deuxième portion 24b soit en contact avec une portion de la pièce de fixation 15.

On retrouve ici les mêmes propriétés physiques que celles décrites ci-dessus pour le premier exemple.

Dans une première variante de réalisation illustrée en figures 6c et 7c, cet élément de positionnement 24 consiste en une languette plate.

Dans une deuxième variante de réalisation illustrée en figures 6b et 7b, cet élément de positionnement 24 consiste en une gouttière apte à couvrir au moins partiellement la zone 2c de l'aiguille 2 et la portion supérieure de la pièce de fixation 15, cette deuxième variante de réalisation permet avantageusement d'éviter le glissement relatif de la pièce de fixation 15 par rapport à l'aiguille 2.

Ainsi, grâce aux différentes caractéristiques décrites ci-dessus, le dispositif 1 selon la présente invention permet de réaliser, de façon simple et sécurisée, la fixation de l'élément de soutènement à l'aiguille d'introduction dans un dispositif pour le traitement de l'incontinence urinaire.

Le dispositif 1 selon la présente invention permet notamment d'éviter le phénomène de ripage constaté dans l'état de la technique.

Il devra être observé que cette description détaillée porte sur deux exemples de réalisation particuliers de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Dispositif (1) pour le traitement de l'incontinence urinaire comprenant une aiguille d'introduction (2) et un élément de soutènement (3) comportant une bandelette et des moyens de fixation aptes à fixer ledit élément de soutènement (3) à l'aiguille d'introduction (2) sur une zone longitudinale déterminée (2c) de celle-ci (2),
a) lesdits moyens de fixation comprennent une pièce de fixation (4, 15), solidaire de l'élément de soutènement (3), constituée au moins partiellement dans un matériau élastiquement déformable et présentant au moins une portion tubulaire (8, 15a, 15b) ayant une ouverture longitudinale (7, 18, 19) agencée en sorte que ladite portion tubulaire (8, 15a, 15b) soit apte à venir s'emboîter sur la zone longitudinale déterminée (2c) de l'aiguille d'introduction (2) par application à force de ladite pièce de fixation (4, 15) selon son ouverture longitudinale sensiblement perpendiculairement ou obliquement à l'aiguille d'introduction (2),
b) la pièce de fixation (4, 15) et l'aiguille d'introduction (2) comportant respectivement des premiers (11, 12) et des seconds (9, 10) moyens d'arrêt aptes à coopérer ensemble pour empêcher le coulissement de la pièce de fixation (4, 15) par rapport à la zone longitudinale déterminée (2c) de l'aiguille d'introduction (2), lorsque ladite pièce de fixation (4, 15) est emboîtée sur cette dite zone longitudinale (2c), **caractérisé en ce que**
c) ledit dispositif comporte en outre un élément de positionnement (24) constitué au moins partiellement dans un matériau élastiquement déformable dont une première portion (24a) couvre une zone (2d) de l'aiguille d'introduction (2) et dont la deuxième portion (24b) est en contact avec la portion supérieure de la pièce de fixation (4, 15), en sorte de faciliter l'emboîtement de ladite pièce de fixation (4, 15) avec l'aiguille d'introduction (2).

2. Dispositif (1) selon la revendication 1, **caractérisé en ce que** l'élément de soutènement (3), éventuellement la bandelette, est fixée sur la face extérieure (8a) de la portion tubulaire (8) de la pièce de fixation (4, 15).

3. Dispositif (1) selon l'une des revendications 1 ou 2, **caractérisé en ce que** l'aiguille d'introduction (2) comporte, dans la zone longitudinale déterminée (2c), un évidement (23) au moins partiellement périphérique dont les bords proximal (9) et distal (10) constituent des seconds moyens d'arrêt pour les faces avant (11) et arrière (12) de la pièce de fixation (4), lesquelles font office de premiers moyens d'arrêt.

4. Dispositif (1) selon la revendication 3, **caractérisé en ce que** l'épaisseur (e) de la portion tubulaire (8) est sensiblement égale à la profondeur (h) de l'évidement (23).

5. Dispositif (1) selon l'une quelconque des revendications 1 à 4, **caractérisé en ce que** la pièce de fixation (4) est constituée d'une seule portion tubulaire (8) dont l'ouverture longitudinale (7) s'étend sur toute la longueur (L1).

6. Dispositif (1) selon la revendication 5, **caractérisé en ce que** la portion tubulaire (8) présente, selon une portion d'extrémité de l'ouverture longitudinale (7), une découpe en biseau (13, 14), ladite découpe étant destinée à venir d'abord en appui sur la zone longitudinale déterminée (2c) de l'aiguille d'introduction (2) pour une application ensuite en oblique de ladite portion tubulaire (8) contre la zone longitudinale déterminée (2c) de l'aiguille d'introduction (2).

7. Dispositif (1) selon l'une quelconque des revendications 1 à 4, **caractérisé en ce que** la pièce de fixation (15) comprend une première (15a) et une seconde (15b) portions tubulaires (15a, 15b), ladite seconde portion tubulaire (15b) venant dans le prolongement de la première portion tubulaire (15a), mais avec son ouverture longitudinale (19) qui est diamétralement opposée à celle (18) de la première portion tubulaire (15a), et **en ce que** la pièce de fixation (15) comporte en outre, de part et d'autre de la jonction (22) entre les deux portions tubulaires (15a, 15b), un orifice (18a, 19a) formé en oblique et dimensionné pour permettre le passage ajusté de l'aiguille d'introduction (2), en sorte que l'emboîtement de la pièce de fixation (15) sur la zone longitudinale déterminée (2c) de l'aiguille d'introduction (2) puisse se faire selon les deux portions tubulaires (15a, 15b) après pivotement de ladite pièce de fixation (15) une fois l'aiguille d'introduction (2) introduite jusqu'à la zone longitudinale déterminée (2c).

8. Dispositif (1) selon la revendication 7, **caractérisé en ce que** la première portion tubulaire (15a) a une longueur (L'1) qui est de deux à trois fois supérieure à la longueur (L'2) de la seconde portion tubulaire (15b).

9. Dispositif (1) selon l'une quelconque des revendications 1 à 8, la zone longitudinale déterminée (2c) de l'aiguille l'introduction (2) ayant une section transversale circulaire de diamètre (D), **caractérisé en ce que** la (ou les) portion tubulaire (8, 15a, 15b) a (ou ont) en section transversale la forme d'un arc de cercle de diamètre (D1) sensiblement égal au diamètre (D) et présentant un angle au centre (A0, A1) faisant de 200° à 320°.

10. Dispositif (1) selon l'une quelconque des revendications 1 à 9, **caractérisé en ce que** la pièce de fixation (4, 15) est constituée au moins partiellement dans l'un des matériaux suivants : polypropylène, polyéthylènetéréphtalate, polyoxyméthylène, polyamide 6, polyamide 66, polysulfone, polyphénylsulfone, polyetheretherketone.

11. Dispositif (1) selon la revendication 1, **caractérisé en ce que** l'élément de positionnement (24) consiste en une languette plate.

12. Dispositif (1) selon la revendication 1, **caractérisé en ce que** l'élément de positionnement (24) consiste en une gouttière apte à couvrir au moins partiellement la zone (2d) de l'aiguille d'introduction (2), et éventuellement la portion supérieure de la pièce de fixation (4, 15), pour éviter le glissement relatif de la pièce de fixation (4, 15) par rapport à l'aiguille d'introduction (2).

## Patentansprüche

1. Vorrichtung (1) zur Behandlung von Harninkontinenz, umfassend eine Einführungsnadel (2) und ein Stützelement (3), umfassend einen Streifen und Befestigungsmittel, die geeignet sind, das Stützelement (3) an der Einführungsnadel (2) auf einer bestimmten Längszone (2c) derselben (2) zu befestigen, wobei:
a) die Befestigungsmittel ein Befestigungsstück (4, 15) umfassen, das mit dem Stützelement (3) verbunden ist, zumindest teilweise aus einem elastisch verformbaren Material gebildet ist und mindestens einen röhrenförmigen Abschnitt (8, 15a,15b) aufweist, mit einer Längsöffnung (7, 18, 19), die derart vorgesehen ist, dass der röhrenförmige Abschnitt (8, 15a, 15b) geeignet ist, auf der bestimmten Längszone (2c) der Einführungsnadel (2) durch festes Andrücken des Befestigungsstücks (4, 15) entlang seiner Längsöffnung im Wesentlichen senkrecht oder schräg zu der Einführungsnadel (2) eingesetzt zu werden,
b) das Befestigungsstück (4, 15) und die Einführungsnadel (2) erste (11, 12) bzw. zweite (9, 10) Haltemittel umfassen, die geeignet sind, zusammenzuwirken, um das Gleiten des Befestigungsstücks (4,15) in Bezug zu der bestimmten Längszone (2c) der Einführungsnadle (2) zu vermeiden, wenn das Befestigungsstück (4, 15) auf dieser Längszone (2c) eingesetzt ist,
**dadurch gekennzeichnet, dass**
c) die Vorrichtung ferner ein Positionierungselement (24) umfasst, das zumindest teilweise aus einem elastisch verformbaren Material hergestellt ist, von dem ein erster Abschnitt (24a) eine Zone (2d) der Einführungsnadel (2) bedeckt, und dessen zweiter Abschnitt (24b) mit dem oberen Abschnitt des Befestigungsstücks (4, 15) in Kontakt ist, um die Einsetzverbindung des Befestigungsstücks (4, 15) mit der Einführungsnadel (2) zu erleichtern.

2. Vorrichtung (1) gemäß Anspruch 1, **dadurch gekennzeichnet, dass** das Stützelement (3), eventuell der Streifen, auf der Außenseite (8a) des röhrenförmigen Abschnitts (8) des Befestigungsstücks (4, 15) befestigt ist.

3. Vorrichtung (1) gemäß einem der Ansprüche 1 oder 2, **dadurch gekennzeichnet, dass** die Einführungsnadel (2) in der bestimmten Längszone (2c) eine zumindest teilweise periphere Ausnehmung (23) umfasst, deren proximalen (9) und distalen (10) Ränder zweite Haltemittel für die vorderen (11) und hinteren (12) Seiten des Befestigungsstücks (4) darstellen, die als erste Haltemittel dienen.

4. Vorrichtung (1) gemäß Anspruch 3, **dadurch gekennzeichnet, dass** die Dicke (e) des röhrenförmigen Abschnitts (8) im Wesentlichen gleich der Tiefe (h) der Ausnehmung (23) ist.

5. Vorrichtung (1) gemäß einem der Ansprüche 1 bis 4, **dadurch gekennzeichnet, dass** das Befestigungsstück (4) von einem einzigen röhrenförmigen Abschnitt (8) gebildet ist, dessen Längsöffnung (7) sich über die gesamte Länge (L1) erstreckt.

6. Vorrichtung (1) gemäß Anspruch 5, **dadurch gekennzeichnet, dass** der röhrenförmige Abschnitt (8) entlang eines Endabschnitts der Längsöffnung (7) einen schrägen Ausschnitt (13,14) aufweist, wobei der Ausschnitt dazu bestimmt ist, zuerst auf der bestimmten Längszone (2c) der Einführungsnadel (2) zur Auflage zu gelangen, um sodann den röhrenförmigen Abschnitt (8) schräg an die bestimmte Längszone (2c) der Einführungsnadel (2) anzulegen.

7. Vorrichtung (1) gemäß einem der Ansprüche 1 bis 4, **dadurch gekennzeichnet, dass** das Befestigungsstück (15) einen ersten (15a) und einen zweiten (15b) röhrenförmigen Abschnitt (15a, 15b) umfasst, wobei der zweite röhrenförmige Abschnitt (15b) in der Verlängerung des ersten röhrenförmigen Abschnitts (15a) angeordnet ist, wobei allerdings seine Längsöffnung (19) diametral zu jener (18) des ersten röhrenförmigen Abschnitts (15a) angeordnet ist, und dass das Befestigungsstück (15) ferner beiderseits der Verbindung (22) zwischen den röhrenförmigen Abschnitten (15a, 15b) eine Öffnung (18a, 19a) umfasst, die schräg ausgebildet und dimensioniert ist, um den angepassten Durchgang der Einführungsnadel (2) zu ermöglichen, so dass das Einsetzen des Befestigungsstücks (15) auf der bestimmten Längszone (2c) der Einführungsnadel (2) entlang der zwei röhrenförmigen Abschnitte (15a, 15b) nach Schwenken des Befestigungsstücks (15) erfolgen kann, wenn die Einführungsnadel (2) bis zu der bestimmten Längszone (2c) eingeführt ist.

8. Vorrichtung (1) gemäß Anspruch 7, **dadurch gekennzeichnet, dass** der ersten röhrenförmige Abschnitt (15a) eine Länge (L'1) hat, die zweimal größer als die Länge (L'2) des zweiten röhrenförmigen Abschnitts (15b) ist.

9. Vorrichtung (1) gemäß einem der Ansprüche 1 bis 8, wobei die bestimmte Längszone (2c) der Einführungsnadel (2) einen kreisförmigen Querschnitt mit einen Durchmesser (D) hat, **dadurch gekennzeichnet, dass** der (oder die) röhrenförmige(n) Abschnitt(e) (8, 15a, 15b) im Querschnitt die Form eines Kreisbogens mit einem Durchmesser (D1) im Wesentlichen gleich dem Durchmesser (D) hat(haben) und einen Winkel in der Mitte (A0, A1) aufweist(en), der 200 ° bis 300 ° bildet.

10. Vorrichtung (1) gemäß einem der Ansprüche 1 bis 9, **dadurch gekennzeichnet, dass** das Befestigungsstück (4, 15) zumindest teilweise aus einem der folgenden Materialien hergestellt ist: Polypropylen, Polyethylenterephthalat, Polyoxymethylen, Polyamid 6, Polyamid 66, Polysulfon, Polyphenylsulfon, Polyetheretherketon.

11. Vorrichtung (1) gemäß Anspruch 1, **dadurch gekennzeichnet, dass** das Positionierungselement (24) in einer flachen Lasche besteht.

12. Vorrichtung (1) gemäß Anspruch 1, **dadurch gekennzeichnet, dass** das Positionierungselement (24) in einer Rinne besteht, die geeignet ist, zumindest teilweise die Zone (2d) der Einführungsnadel (2) und eventuell den oberen Abschnitt des Befestigungsstücks (4, 15) zu bedecken, um das relative Gleiten des Befestigungsstücks (4, 15) in Bezug zur Einführungsnadel (2) zu vermeiden.

## Claims

1. A device (1) for treating urinary incontinence comprising an insertion needle (2) and a retaining element (3) including a strip and fastening means able to fasten said retaining element (3) to the insertion needle (2) over a determined longitudinal zone (2c) thereof (2),
a) said fastening means comprising a fastening part (4, 15), secured to the retaining element (3), made up at least partially of an elastically deformable material and having at least one tubular portion (8, 15a, 15b) having a longitudinal opening (7, 18, 19) arranged such that said tubular portion (8, 15a, 15b) is able to nest on the determined longitudinal zone (2c) of the insertion needle (2) by applying force to said fastening part (4, 15) along its longitudinal opening substantially perpendicularly or obliquely with respect to the insertion needle (2),
b) the fastening part (4, 15) and the insertion needle (2) respectively including first (11, 12) and second (9, 10) stop means able to cooperate with one another to prevent the fastening part (4, 15) from sliding relative to the determined longitudinal zone (2c) of the insertion needle (2), when said fastening part (4, 15) is nested on this said longitudinal zone (2c), **characterized in that**
c) said device further includes a positioning element (24) made up at least partially of an elastically deformable material whereof a first portion (24a) covers a zone (2d) of the insertion needle (2) and whereof the second portion (24b) is in contact with the upper portion of the fastening part (4, 15), so as to facilitate the nesting of said fastening part (4, 15) with the insertion needle (2).

2. The device (1) according to claim 1, **characterized in that** the retaining element (3), optionally the strip, is fastened on the outer face (8a) of the tubular portion (8) of the fastening part (4, 15).

3. The device (1) according to one of claims 1 or 2, **characterized in that** the insertion needle (2) includes, in the determined longitudinal zone (2c), an at least partially peripheral recess (23) whereof the proximal (9) and distal (10) edges constitute second stop means for the front (11) and rear (12) faces of the fastening part (4), which serve as first stop means.

4. The device (1) according to claim 3, **characterized in that** the thickness (e) of the tubular portion (8) is substantially equal to the depth (h) of the recess (23).

5. The device (1) according to any one of claims 1 to 4, **characterized in that** the fastening part (4) is made up of a single tubular portion (8) whose longitudinal opening (7) extends over the entire length (L1).

6. The device (1) according to claim 5, **characterized in that** the tubular portion (8) has, along an end portion of the longitudinal opening (7), a beveled cutout (13, 14), said cutout being intended first to bear on the determined longitudinal zone (2c) of the insertion needle (2) for a subsequent oblique application of the tubular portion (8) against the determined longitudinal zone (2c) of the insertion needle (2).

7. The device (1) according to any one of claims 1 to 4, **characterized in that** the fastening part (15) comprises a first (15a) and second (15b) tubular portion (15a, 15b), said second tubular portion (15b) being in the extension of the first tubular portion (15a), but with its longitudinal opening (19) diametrically opposite that (18) of the first tubular portion (15a), and **in that** the fastening part (15) further includes, on both sides of the junction (22) between the two tubular portions (15a, 15b), an oblique orifice (18a, 19a) dimensioned to allow the adjusted passage of the insertion needle (2), such that the nesting of the fastening part (15) on the determined longitudinal zone (2c) of the insertion needle (2) can be done along the two tubular portions (15a, 15b) after pivoting of said fastening part (15) once the insertion needle (2) is inserted up to be determined longitudinal zone (2c).

8. The device (1) according to claim 7, **characterized in** the first tubular portion (15a) has a length (L'1) that is 2 to 3 times greater than the length (L'2) of the second tubular portion (15b).

9. The device (1) according to any one of claims 1 to 8, the determined longitudinal zone (2c) of the insertion needle (2) having a circular cross-section with diameter (D), **characterized in that** the tubular portion(s) (8, 15a, 15b) has (or have), in cross-section, an arc of circle shape with a diameter (D1) substantially equal to the diameter (D) and having a center angle (A0, A1) from 200° to 320°.

10. The device (1) according to any one of claims 1 to 9, **characterized in that** the fastening part (4, 15) is made up at least partially from one of the following materials: polypropylene, polyethylene terephthalate, polyoxyethylene, polyamide 6, polyamide 66, polysulfone, polyphenol sulfone, polyether ether ketone.

11. The device (1) according to claim 1, **characterized in that** the positioning element (24) consists of a flat tongue.

12. The device (1) according to claim 1, **characterized in that** the positioning element (24) consists of a gutter able to at least partially cover the zone (2d) of the insertion needle (2), and optionally the upper portion of the fastening part (4, 15), to prevent the relative sliding of the fastening part (4, 15) with respect to the insertion needle (2).
